# FASCICULE DE BREVET EUROPEEN

(11) **EP 0 986 519 B1**
(45) Date de publication et mention de la délivrance du brevet: **12.12.2001**
(21) Numéro de dépôt: 98923942.1
(22) Date de dépôt: 05.06.1998
(51) Int. Cl.: C02F 1/68, C02F 3/34, C11D 3/386, C11D 11/00, C12N 11/00, B08B 9/02

(54) **PRODUIT BIOLOGIQUE POUR L'ENTRETIEN DES CANALISATIONS ET POUR L'EPURATION DES EAUX USEES**
BIOLOGISCHES PRODUKT ZUR INSTANDHALTUNG VON ROHRLEITUNGEN UND ZUR ABWASSERREINIGUNG
BIOLOGICAL PRODUCT FOR MAINTAINING WATER MAINS AND PURIFYING WASTE WATER

(30) Priorité: 05.06.1997 WO PCT/BE97/00065
(43) Date de publication de la demande: 22.03.2000
(73) Titulaire: Bevil SPRL, 1300 Wavre (BE)
(72) Inventeur: KNAPEN, Guy, B-1560 Hoeilaart (BE)
(74) Mandataire: Vandeberg, Marie-Paule L.G.
(86) Numéro de dépôt international: BE9800081
(87) Numéro de publication internationale: WO9855406

(56) Documents cités:
- FR-A- 2 622 206
- US-A- 3 242 055
- US-A- 3 506 582
- US-A- 3 798 181
- US-A- 4 670 149
- US-A- 4 810 385
- US-A- 5 275 943
- US-A- 5 543 309
- US-A- 5 630 883

## Description

### Domaine technique

La présente invention concerne un produit biologique pour l'épuration des eaux usées, en particulier pour l'épuration des eaux ménagères, particulièrement adapté pour l'entretien de canalisations, notamment de canalisations à siphon, telles que des canalisations d'éviers, de douches et de baignoires, ainsi qu'un procédé pour l'entretien de canalisations et un procédé pour l'épuration d'eaux usées.

### Art antérieur et critique de cet art antérieur

On connaît de nombreuses compositions biologiques utilisées dans diverses circonstances pour l'épuration des eaux usées. Par "compositions biologiques", on entend des compositions comprenant des micro-organismes et/ou des enzymes.

Différents conditionnements de telles compositions biologiques sont actuellement présents sur le marché. Les compositions biologiques connues se présentent généralement sous forme liquide ou sous forme de poudre, le cas échéant comprimée. Elles peuvent également se présenter sous forme de granules. De telles compositions, qui peuvent aussi être effervescentes et comprendre des détergents, exercent le plus fréquemment une action temporaire de nettoyage, par exemple pour l'entretien de canalisations, telles que, entre autres, des canalisations d'éviers. Des exemples de telles compositions sont décrits notamment dans les documents FR 2 622 206, US 3 506 582, WO 96/13342, et DE 3 906 124.

Il est également connu d'utiliser des compositions biologiques pour l'épuration des eaux usées, ces compositions se retrouvant sur différents supports solides peu ou pas dégradables, destinés à rester en place dans des sites recevant en continu des matières organiques à dégrader, par exemple dans des bioréacteurs et collecteurs. De telles compositions sont mentionnées dans les documents US 4 810 385 et US 4 670 149.

### But de l'invention

La présente invention a pour but de proposer un produit biologique pour l'épuration des eaux usées, d'utilisation simple et efficace, susceptible d'exercer une action continue, spécialement bien adapté pour l'entretien en continu des canalisations, notamment des canalisations d'éviers de cuisine et de salle de bain, sans toutefois exclure d'autres applications.

### Description

L'invention a pour objet un produit biologique qui comporte un bâtonnet comprimé comportant au moins une espèce bactérienne, au moins une enzyme apte à permettre l'hydrolyse de matières organiques, et au moins un agent de compression. Le bâtonnet peut être muni de tout accessoire facilitant sa manipulation.

Ce produit biologique peut être avantageusement utilisé pour l'épuration des eaux usées et l'entretien de canalisations.

Il est intéressant d'utiliser des espèces bactériennes thermiquement résistantes, par exemple des espèces bactériennes sous forme de spores. Celles-ci sont dormantes au moment de la fabrication des bâtonnets, et protégées d'un point de vue thermique, ce qui est un avantage non négligeable par exemple lors d'un processus d'extrusion à chaud et également lors du contact du bâtonnet avec des produits chauds, par exemple déversés dans des canalisations.

La ou les espèce(s) bactérienne(s) peut ou peuvent être choisie(s) parmi les espèces suivantes : *Bacillus pumilus*, *Bacillus subtilis, Bacillus megaterium*, *Bacillus polymixa,* et les combinaisons de ces espèces. De manière préférée, ces quatre espèces sont présentes dans le bâtonnet.

La ou les enzyme(s) peut ou peuvent être choisie(s) parmi la lipase, l'amylase et la protéase et les combinaisons de ces enzymes. De manière préférée, ces trois enzymes sont présentes dans le bâtonnet. On peut utiliser par exemple de la lipase produite par *Aspergillus oryzea*, de l'amylase produite par *Bacillus amyloliquefaciens*, et de la protéase produite par *Bacillus licheniformis*.

Dans une forme d'exécution préférée, le bâtonnet est extrudé.

Les produits biologiques de l'invention peuvent comporter en outre au moins une substance nutritive. Les substances nutritives peuvent être choisies parmi les sources de protides, de glucides et de lipides et leurs combinaisons. De manière préférée, toutes ces sources sont présentes dans le bâtonnet.

Dans une forme d'exécution spécifique, particulièrement utile pour l'utilisation dans des canalisations de salle de bain, ils peuvent comprendre en outre au moins un détergent tel que le laurylsulfate de sodium.

Le(s) agent(s) de compression peuvent comprendre un mélange de PVC, d'éthylcellulose, et d'amidon. Les agents de compression sont progressivement désagrégés après contact avec l'eau, ce qui permet avantageusement une libération progressive et continue des principes actifs du bâtonnet.

Suivant une forme d'exécution particulière, les produits biologiques de l'invention peuvent comporter de 0,5 à 2 % en poids d'espèce(s) bactérienne(s) provenant d'une poudre à une concentration de 1,1 10¹¹ spores/g. Ils peuvent comporter de 5 à 20 % en poids d'enzyme(s).

Les bâtonnets peuvent présenter des formes diverses et ne sont pas nécessairement rectilignes. Ils peuvent présenter une section transversale choisie parmi les sections transversales circulaires, quasi-circulaires, elliptiques, et les sections polygonales. Ils ont de préférence une longueur comprise entre 5 et 10 cm.

L'invention a également pour objet un procédé pour l'entretien de canalisations, dans lequel on introduit un produit biologique suivant l'invention dans un siphon de canalisation, et un procédé pour l'épuration d'eaux usées, dans lequel on introduit un produit biologique suivant l'invention dans un dispositif d'épuration.

### Description détaillée

D'autres caractéristiques et avantages de l'invention apparaîtront à la lecture de la description qui suit d'un exemple de réalisation. On décrit ci-dessous la fabrication d'un produit biologique en bâtonnet extrudé suivant l'invention, qui peut être utilisé avantageusement dans une canalisation d'évier de cuisine.

On commence par réaliser le mêlange de base pour la fabrication du bâtonnet, qui est constitué majoritairement d'agents de compression. On solubilise d'abord 8 % en poids d'éthylcellulose à l'aide de méthanol. Le méthanol s'évapore et n'intervient donc pas pour le calcul du poids final du mélange à extruder. On mélange 75 % en poids de PVC, et 6 % en poids de dérivès d'amidon avec l'éthylcellulose solubilisée. On ajoute 0,01 % de colorants. On homogénéise le mélange ainsi obtenu quelques minutes sur un agitateur planétaire pour obtenir une granulation. En fin de processus, on ajoute 1 % en poids d'une poudre comprenant les 4 espèces bactériennes suivantes : *Bacillus pumilus*, *Bacillus subtilis Bacillus megaterium et Bacillus polymixa,* cette poudre comportant 1,1 10¹¹ spores/g, et 10 % en poids d'une poudre contenant 60 % de lipase, 20 % d'amylase et 20 % de protéase. Lorsque la granulation est terminée, on procède à une extrusion classique, au terme de laquelle on obtient un bâtonnet dont la section, le diamètre et la longueur peuvent être réglés en fonction des désirs du fabricant Pour l'utilisation dans des éviers de cuisine à siphons, la fabrication de bâtonnets de section cylindrique, d'un diamètre de 0,5 cm et d'une longueur de 10 cm donne des résultats satisfaisants. Il est clair que l'on peut adapter cette forme et ces dimensions à toute application spécifique pour obtenir l'effet technique souhaité.

Les produits biologiques sous forme de bâtonnets de l'invention (également désignés dans le présent texte sous l'appellation de "bâtonnets suivant l'invention") sont destinés à être commercialisés à l'état sec, et présentés sous une forme appropriée, par exemple sous blister. Aussi longtemps que le bâtonnet est au sec, il conserve sa composition originale. Les espèces bactériennes et les enzymes sont inactives et il n'y a pas de risque de dégradation interne.

Les bâtonnets suivant l'invention peuvent être utilisés pour l'épuration d'eaux usées aussi bien dans des canalisations que dans d'autres applications. Dans tous les cas, leur mise en place est extrêmement simple.

Dans le cas de l'utilisation des bâtonnets dans une canalisation d'évier, il suffit de laisser tomber d'un simple geste un bâtonnet dans la crépine de l'évier. Par gravité, il va se positionner dans le siphon, dans lequel il reste coincé grâce à sa forme et à ses dimensions.

L'humidification des bâtonnets en place est graduelle, et leur composition spécifique utilisée tient compte de cet élément. Après humidification, les principes actifs produits par le bâtonnet sont libérés dans le siphon de la canalisation de manière progressive et efficace.

Les bâtonnets de l'invention sont efficaces dès leur mise en place et tout au cours de leur utilisation.

Les enzymes présentes à l'origine dans le bâtonnet commencent à exercer leur action sur les matières organiques contenues dans les premières eaux usées passant dans le siphon après la mise en place du bâtonnet dans celui-ci. Lors du passage de ces premières eaux, les substances nutritives sont humidifiées, et les espèces bactériennes progressivement activées. Ces dernières, une fois leur état de dormance levé, sont elles-mêmes capables de produire des enzymes qui seront aptes à prendre le relais des enzymes présentes au départ dans le bâtonnet.

Différentes enzymes peuvent être produites par les différentes espèces bactériennes : ainsi, *Bacillus pumilus* peut produire de la cellulase, de la lichenase, de la sérine protéase, de la sérine métal protéase ; *Bacillus subtilis* peut produire de l'alpha amylase, de la métal protéase, de la sérine protéase ; *Bacillus megaterium* peut produire de la bêta amylase et de la métal protéase ; *Bacillus polymixa* peut produire de la bêta amylase, de l'iso amylase, de la métal protéase et de la xylanase.

Les agents de compression utilisés se dégradent au fur et à mesure de l'humidification du bâtonnet, qui s'érode progressivement et finit par disparaître.

II importe de noter que la composition des bâtonnets suivant l'invention est telle que pendant la période d'utilisation dans la canalisation qui est visée se déroule un programme séquentiel qui permet d'obtenir en permanence et pour une durée souhaitée une bonne efficacité du produit.

En fonction de la taille et du poids des bâtonnets, ainsi que de la fréquence d'utilisation des canalisations, la durée moyenne d'utilisation peut varier. Ainsi, par exemple, un bâtonnet d'une longueur de 10 cm et d'un diamètre de 0,5 cm pesant environ 4 g et contenant 1,1 10⁹ spores durera environ 1 mois si on l'utilise dans un évier de cuisine d'une capacité de 20 litres servant en moyenne 3 fois par jour.

L'efficacité des tels bâtonnets de l'invention a été testée de la façon suivante : deux récipients de 100 ml sont remplis d'eau, à laquelle on ajoute des croquettes pour animaux contenant 12% de chair de poulet, 10% de foie de poulet, des extraits de protéines végétales, des substances minérales, des extraits de poisson, des huiles, graisses, sucres et extraits de légumes. Un bâtonnet est placé avant la nuit dans un des deux récipients. Au matin suivant, le récipient témoin dégage un odeur nauséabonde et les croquettes sont faiblement dégradées. Le récipient traité par le bâtonnet de l'invention est inodore, et la matière qui constituait les croquettes y est liquéfiée. L'essai est répété 31 fois avec le même bâtonnet pour vérifier la continuité de l'efficacité durant un mois.

II faut noter que les éléments constitutifs du bâtonnet finissent, lorsque celui-ci est en place dans un siphon, par être entraînés et progressivement éliminés, d'où l'intérêt de mettre en place un second bâtonnet avant la complète disparition du premier.

Comme les bâtonnets se mettent en place dans le siphon (opaque), il n'est pas envisagé de contrôler s'ils sont ou non encore présents après un certain temps d'utilisation. Ceci serait d'ailleurs inutile car la mise en place d'un second bâtonnet peut être faite sans problème au bout d'une période moyenne prédéterminée. Si le bâtonnet précédent n'a pas encore totalement disparu, les deux bâtonnets peuvent coexister sans gêne l'un pour l'autre

Les produits biologiques suivant l'invention sont extrêmement faciles à utiliser. Ils sont non corrosifs.

Ni pour la mise en place du premier bâtonnet, ni lors de l'installation des suivants dans une canalisation, il n'est nécessaire de procéder à un quelconque démontage.

On comprendra que les exemples ci dessus ne sont pas limitatifs, et que de nombreuses variantes peuvent être conçues sans sortir du cadre de la présente invention. En particulier, diverses formes et dimensions peuvent être utilisées.

Bien que les produits de l'invention soient particulièrement bien adaptés pour leur utilisation dans des canalisations, il est clair qu'on peut également les utiliser par exemple pour l'ensemencement de fosses septiques ou de bacs à graisses ou dans toute autre application similaire.

## Revendications

1. Produit biologique, **caractérisé en ce qu'**il comporte un bâtonnet comprimé comportant les éléments suivants
- au moins une espèce bactérienne ;
- au moins une enzyme apte à permettre l'hydrolyse de matières organiques, et
- au moins un agent de compression.

2. Produit biologique suivant la revendication 1, **caractérisé en ce que** l'espèce bactérienne est présente sous forme de spores.

3. Produit biologique suivant l'une quelconque des revendications 1 et 2, **caractérisé en ce que** le bâtonnet est extrudé.

4. Produit biologique suivant l'une quelconque des revendications 1 à 3, **caractérisé en ce qu'**il comporte en outre au moins une substance nutritive.

5. Produit biologique suivant l'une quelconque des revendications précédentes, **caractérisé en ce qu'**il comporte en outre au moins un détergent.

6. Produit biologique suivant l'une quelconque des revendications précédentes, **caractérisé en ce que** la au moins une espèce bactérienne est choisie parmi les espèces suivantes : *Bacillus pumilus*, *Bacillus subtilis*, *Bacillus megaterium*, *Bacillus polymixa*, et les combinaisons de ces espèces.

7. Produit biologique suivant. l'une quelconque des revendications précédentes, **caractérisé en ce que** la au moins une enzyme est choisie parmi la lipase, l'amylase et la protéase et les combinaisons de ces enzymes.

8. Produit biologique suivant la revendication 7 comportant de la lipase, **caractérisé en ce que** la lipase est de la lipase produite par *Aspergillus oryzea.*

9. Produit biologique suivant l'une quelconque des revendications 7 et 8 comportant de l'amylase, **caractérisé en ce que** l'amylase est de l'amylase produite par *Bacillus amyloliquefaciens.*

10. Produit biologique suivant l'une quelconque des revendications 7 à 9 comportant de la protéase, **caractérisé en ce que** la protéase est de la protéase produite par *Bacillus licheniformis.*

11. Produit biologique suivant l'une quelconque des revendications précédentes, **caractérisé en ce que** la au moins un agent de compression comprend un mélange de PVC, d'éthylcellulose, et d'amidon.

12. Produit biologique suivant l'une quelconque des revendications 4 à 11, **caractérisé en ce que** les substances nutritives sont choisies parmi les sources de protides, de glucides et de lipides et leurs combinaisons.

13. Produit biologique suivant l'une quelconque des revendications 5 à 12, **caractérisé en ce que** le détergent est le laurylsulfate de sodium.

14. Produit biologique suivant l'une quelconque des revendications précédentes, **caractérisé en ce qu'**il comporte 0,5 à 2 % en poids d'espèce(s) bactérienne(s) provenant d'une poudre à une concentration de 1,1 10 ¹¹ spores/g

15. Produit biologique suivant l'une quelconque des revendications précédentes, **caractérisé en ce qu'**il comporte de 5 à 20 % en poids d'enzyme(s).

16. Produit biologique suivant l'une quelconque des revendications précédentes, **caractérisé en ce que** le bâtonnet présente une section transversale choisie parmi les sections transversales circulaires, quasi-circulaires, elliptiques, et les sections polygonales.

17. Produit biologique suivant l'une quelconque des revendications précédentes, **caractérisé en ce que** le bâtonnet présente une longueur comprise entre 5 et 10 cm.

18. Procédé pour l'entretien de canalisations, **caractérisé en ce que** l'on introduit un produit biologique suivant l'une quelconque des revendications 1 à 17 dans un siphon de canalisation.

19. Procédé pour l'épuration d'eaux usées, **caractérisé en ce que** l'on introduit un produit biologique suivant l'une quelconque des revendications 1 à 17 dans un dispositif d'épuration.

## Patentansprüche

1. Biologisches Erzeugnis, **dadurch gekennzeichnet, daß** es einen verdichteten Stab mit den folgenden Elementen umfaßt:
- zumindest eine Bakterienart,
- zumindest ein Enzym, das geeignet ist, eine Hydrolyse organischer Stoffe zu ermöglichen, und
- zumindest ein Verdichtungsmittel.

2. Biologisches Erzeugnis nach Anspruch 1, **dadurch gekennzeichnet, daß** die Bakterienart in Form von Sporen vorhanden ist.

3. Biologisches Erzeugnis nach einem der Ansprüche 1 und 2, **dadurch gekennzeichnet, daß** der Stab extrudiert ist.

4. Biologisches Erzeugnis nach einem der Ansprüche 1 bis 3, **dadurch gekennzeichnet, daß** es außerdem zumindest einen Nährstoff enthält.

5. Biologisches Erzeugnis nach einem der vorhergehenden Ansprüche, **dadurch gekennzeichnet, daß** es außerdem ein Detergens umfaßt.

6. Biologisches Erzeugnis nach einem der vorhergehenden Ansprüche, **dadurch gekennzeichnet, daß** die zumindest eine Bakterienart aus den folgenden Bakterienarten ausgewählt ist: *Bacillus pumilus, Bacillus subtilis*, *Bacillus megaterium*, *Bacillus polymixa* und den Kombinationen dieser Arten.

7. Biologisches Erzeugnis nach einem der vorhergehenden Ansprüche, **dadurch gekennzeichnet, daß** das zumindest eine Enzym ausgewählt ist aus Lipase, Amylase und Protease und Kombinationen dieser Enzyme.

8. Biologisches Erzeugnis nach Anspruch 7, Lipase enthaltend, **dadurch gekennzeichnet, daß** die Lipase eine von *Aspergillus oryzea* erzeugte Lipase ist.

9. Biologisches Erzeugnis nach einem der Ansprüche 7 und 8, Amylase enthaltend, **dadurch gekennzeichnet, daß** die Amylase eine von *Bacillus amyloliquefaciens* erzeugte Amylase ist.

10. Biologisches Erzeugnis nach einem der Ansprüche 7 bis 9, Protease umfassend, **dadurch gekennzeichnet, daß** die Protease eine von *Bacillus licheniformis* erzeugte Protease ist.

11. Biologisches Erzeugnis nach einem der vorhergehenden Ansprüche, **dadurch gekennzeichnet, daß** das zumindest eine Verdichtungsmittel eine Mischung von PVC, Ethylcellulose und Stärke ist.

12. Biologisches Erzeugnis nach einem der Ansprüche 4 bis 11, **dadurch gekennzeichnet, daß** die Nährstoffe aus Quellen von Proteinen, Zucker und Lipiden und deren Kombinationen ausgewählt sind.

13. Biologisches Erzeugnis nach einem der Ansprüche 5 bis 12, **dadurch gekennzeichnet, daß** das Detergens Natriumlaurylsulfat ist.

14. Biologisches Erzeugnis nach einem der vorhergehenden Ansprüche, **dadurch gekennzeichnet, daß** es 0,5 bis 2 Gew.-% Bakterienart(en) aus einem Pulver mit einer Konzentration von 1,1 10¹¹ Sporen/g umfaßt.

15. Biologisches Erzeugnis nach einem der vorhergehenden Ansprüche, **dadurch gekennzeichnet, daß** es 5 bis 20 Gew.-% Enzym(e) umfaßt.

16. Biologisches Erzeugnis nach einem der vorhergehenden Ansprüche, **dadurch gekennzeichnet, daß** der Stab einen Querschnitt aufweist, der aus kreisförmigen, annähernd kreisförmigen, elliptischen und polygonalen Querschnitten ausgewählt ist.

17. Biologisches Erzeugnis nach einem der vorhergehenden Ansprüche, **dadurch gekennzeichnet, daß** der Stab eine Länge von 5 bis 10 cm aufweist.

18. Verfahren zur Wartung von Abwasserleitungen, **dadurch gekennzeichnet, daß** man ein biologisches Erzeugnis nach einem der Ansprüche 1 bis 17 in einen Abwasserleitungs-Siphon einsetzt.

19. Verfahren zur Reinigung von Abwasser, **dadurch gekennzeichnet, daß** man ein biologisches Erzeugnis nach einem der Ansprüche 1 bis 17 in eine Reinigungsvorrichtung einsetzt.

## Claims

1. Biological product, **characterized in that** it comprises a compressed stick comprising the following components
- at least one bacterial species;
- at least one enzyme capable of allowing the hydrolysis of organic materials, and
- at least one compressing agent.

2. Biological product according to Claim 1, **characterized in that** the bacterial species is present in the form of spores.

3. Biological product according to either of Claims 1 and 2, **characterized in that** the stick is extruded.

4. Biological product according to any one of Claims 1 to 3, **characterized in that** it also comprises at least one nutrient substance.

5. Biological product according to any one of the preceding claims, **characterized in that** it also comprises at least one detergent.

6. Biological product according to any one of the preceding claims, **characterized in that** the at least one bacterial species is chosen from the following species: *Bacillus pumilus*, *Bacillus subtilis*, *Bacillus megaterium*, *Bacillus polymixa,* and combinations of these species.

7. Biological product according to any one of the preceding claims, **characterized in that** the at least one enzyme is chosen from lipase, amylase and protease, and combinations of these enzymes.

8. Biological product according to Claim 7, comprising lipase, **characterized in that** the lipase is lipase produced by *Aspergillus oryzea.*

9. Biological product according to either of Claims 7 and 8, comprising amylase, **characterized in that** the amylase is amylase produced by *Bacillus amyloliquefaciens.*

10. Biological product according to any one of Claims 7 to 9, comprising protease, **characterized in that** the protease is protease produced by *Bacillus licheniformis.*

11. Biological product according to any one of the preceding claims, **characterized in that** the at least one compressing agent comprises a mixture of PVC, ethylcellulose and starch.

12. Biological product according to any one of Claims 4 to 11, **characterized in that** the nutrient substances are chosen from sources of proteins, carbohydrates and lipids, and combinations thereof.

13. Biological product according to any one of Claims 5 to 12, **characterized in that** the detergent is sodium lauryl sulphate.

14. Biological product according to any one of the preceding claims, **characterized in that** it comprises 0.5 to 2% by weight of bacterial species originating from a powder at a concentration of 1.1 × 10¹¹ spores/g.

15. Biological product according to any one of the preceding claims, **characterized in that** it comprises from 5 to 20% by weight of enzyme(s).

16. Biological product according to any one of the preceding claims, **characterized in that** the stick has a transverse cross section chosen from circular, virtually circular and elliptical transverse cross sections, and polygonal cross sections.

17. Biological product according to any one of the preceding claims, **characterized in that** the stick is between 5 and 10 cm in length.

18. Process for maintaining pipes, **characterized in that** a biological product according to any one of Claims 1 to 17 is introduced into a pipe siphon.

19. Process for purifying waste water, **characterized in that** a biological product according to any one of Claims 1 to 17 is introduced into a purification device.
